# EUROPEAN PATENT SPECIFICATION

(11) **EP 0 723 957 B1**
(45) Date of publication and mention of the grant of the patent: **21.05.2003**
(21) Application number: 95309452.1
(22) Date of filing: 27.12.1995
(51) Int. Cl.: C07D 207/28, A61K 31/40, C07D 401/12

(54) **Lactacystin derivatives**
Lactacystinderivate
Dérivés de lactacystin

(30) Priority: 27.12.1994 JP 32446594
(43) Date of publication of application: 31.07.1996
(73) Proprietor: THE KITASATO INSTITUTE, Minato-ku Tokyo (JP)
(72) Inventor: Omura, Satoshi, c/o The Kitasato Institute, Tokyo (JP); Sunazuka, Toshiaki, c/o The Kitasato Institute, Tokyo (JP); Tanaka, Haruo, c/o The Kitasato Institute, Tokyo (JP)
(74) Representative: Woods, Geoffrey Corlett

(56) References cited:
- J. ANTIBIOT. (1995), 48(7), 747-8 CODEN: JANTAJ;ISSN: 0021-8820, July 1995, XP002000833 NAGAMITSU, TOHRU ET AL: "Structure-activity relationships of lactacystin, the first non-protein neurotrophic factor"
- TETRAHEDRON LETT. (1993), 34(44), 6977-80 CODEN: TELEAY;ISSN: 0040-4039, 1993, XP002000834 COREY, E. J. ET AL: "Studies on the total synthesis of lactacystin. An improved aldol coupling reaction and a.beta.-lactone intermediate in thiol ester formation"
- TETRAHEDRON LETT. (1993), 34(44), 6973-6 CODEN: TELEAY;ISSN: 0040-4039, 1993, XP002000835 COREY, E. J. ET AL: "Synthesis of (6R,7S)-lactacystin and 6-deoxylactacystin from a common intermediate"
- TETRAHEDRON LETT. (1993), 34(44), 6969-72 CODEN: TELEAY;ISSN: 0040-4039, 1993, XP002000836 COREY, E. J. ET AL: "An enantioselective synthesis of (6R)-lactacystin"
- SCIENCE, vol. 268, 5 May 1995, pages 726-731, XP002000837
- PROC. NATL. ACAD. SCI. USA, vol. 91, no. 8, 1994, pages 3358-3362, XP002000838

## Description

This invention relates to lactacystin derivatives, which are derived from lactacystin produced by a microorganism strain Streptomyces sp. OM-6519 belonging to genus Streptomyces with inducing outgrowth of neurites. The compounds induce outgrowth of neurite, with low cytotoxicity and high selective toxicity and are useful for pharmaceuticals.

It has been known that lactacystin of the formula 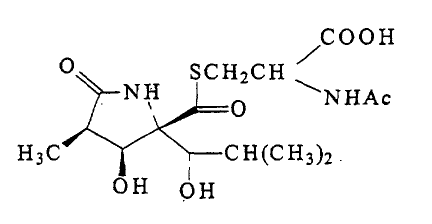 having neuritogenesis activity was produced by culturing physiologically active substance OM-6519 producing microorganism belonging to genus Streptomyces and isolating the compound hereinabove. (J. Antibiotics. 19: 44, 113-116 and Japan. Pat. Unexam. Publ., No 3-98594).

Nagamitsu, J. Antibiotics (1995) Vol. 48,7,747-748, Fenteany *et al,* Science (1995) vol 268,726-731, and Fenteany *et al,* Proc. Natl. Acad. Sci (1994) Vol. 91, 3358-3362, all describe lactacystin or lactacystin derivatives and the activity of those compounds. Corey *et al,* Tetrahedron letters 1993 Vol. 34, 44, 6977-6980, Corey *et al,* Tetrahedron letters 1993 Vol. 34, 44, 6973-6976, and Corey *et al*, Tetrahedron letters 1993 Vol. 34, 44, 6969-6972, describe synthesis of lactacystin.

Lactacystin has the property of specifically inducing activity of outgrowth of neurite, however it is highly cytotoxic and shows no selective toxicity. Accordingly, lactacystin derivatives with superior inducing activity of neuritogenesis and high selective cytotoxicity have been desired to find out.

We have sought to solve the above problems and found that lactacystin derivatives of the formula (1) hereinbelow showed superior inducing activity of neuritogenesis and high selective toxicity to the known lactacystin.

The present invention provides a lactacystin derivative of the formula 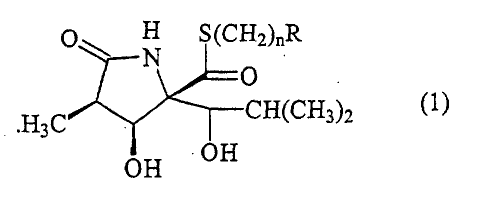 wherein R is branched or unbranched C₁-C₄ alkyl, and
n is 0 to 4, or a pharmacologically acceptable salt thereof.

Examples of alkyl are methyl, ethyl, propyl, isopropyl, butyl, isobutyl, s-butyl and t-butyl. Preferable examples are methyl, ethyl, propyl and isopropyl.

A derivative of formula(1) can be produced by reacting β-lactone (2) of the formula (2): 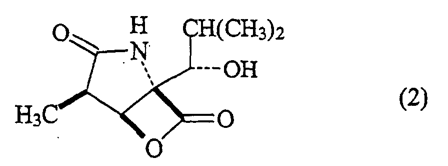 with a mercapto compound of the formula

HS-(CH₂)ₙR

wherein n and R are as defined above, in the presence of a tertiary organic amine in an inert organic solvent.

Examples of the inert organic solvent used in the above reaction are dichloromethane, chloroform and tetrahydrofuran. Examples of the tertiary organic amine used in the above reaction are known tertiary organic amines. Preferred examples are trimethylamine and triethylamine.

Preferred examples of the mercapto compound are, for example, ethane thiol, propane thiol, isopropane thiol, butane thiol, 1-methyl-1-propane thiol and 1-pentane thiol.

The above reaction proceeds preferably under inert gas such as argon and nitrogen gas. The reaction proceeds at room temperature. Reaction can be traced by TLC and HPLC, and is terminated by maximum production of the desired derivative.

Isolation of the derivative can be performed by removing solvent from the reaction mixture and treating the residue with column chromatography.

The thus obtained derivative (1) can be further purified by means of conventional means for isolation and purification of organic compounds, for example combination of extraction, crystallization and chromatography.

Pharmacologically acceptable salts of the derivative (1) can be prepared by conventional methods for preparation of the salt.

Examples of the derivatives of the present invention or comparison compounds are as follows:
3-hydroxy-2-(1-hydroxy-2-methylpropyl)-4-methyl-5-oxo-2-pyrrolidine-ethanethiocarboxylate (1)

   R: CH₃, n = 1
3-hydroxy-2-(1-hydroxy-2-methylpropyl)-4-methyl-5-oxo-2-pyrrolidine-propanethiocarboxylate (2)

   R: CH₃, n=2
3-hydroxy-2-(1-hydroxy-2-methylpropyl)-4-methyl-5-oxo-2-pyrrolidine-iso-propanethiocarboxylate (3)

   R: -CH(CH₃)₂, n = 0
3-hydroxy-2-(1-hydroxy-2-methylpropyl)-4-methyl-5-oxo-2-pyrrolidine-butanethiocarboxylate (4)

   R: -(CH₂)₃CH₃, n = 0
3-hydroxy-2-(1-hydroxy-2-methylpropyl)-4-methyl-5-oxo-2-pyrrolidine-1-methyl-1-propanethiocarboxylate (5)

   R: -CH₂CH(CH₃)₂, n = 0
3-hydroxy-2-(1-hydroxy-2-methylpropyl)-4-methyl-5-oxo-2-pyrrolidine-1-pentanethiocarboxylate (6)

   R : CH₃, n = 4

Pharmacological activity of the derivative (1) and its pharmacologically acceptable salt are explained hereinbelow.

### 1. Activity of neuritogenesis and cytotoxic action:

### (1) Test method:

Morphological changes of Neuro 2A cells are obseved according to the method of Baglioni et al. [J. Biol Chem., 266: 18620 (1991)].

Neuro 2A cells were plated at a density of 1 × 10 ⁴ cells/cm² in a 24 hole plate, and cultured in MEM-H with 10% FBS. On the next day, lactacystin or lactacystin derivatives were added at the concentration of 0.05 - 100 µM, and morphological changes of the cells were observed time dependently by- phase contrast microscope.

Minimum effective dose of the substances was defined as a concentration in which approximately 20% of whole cells showed induction of bipolar outgrowth of neurite.

Cytotoxicity of the compounds is defined when no attached Neuro 2A cells were observed at above 2/3 of the whole cells.

### (2) Results:

Results of the tests are shown in Table 1.

### 2. No specific test method for selective toxicity of the compounds is known, therefore the selective toxicity is defined as a ratio of cytotoxicity/minimum effective dose.

Result is shown in Table 1.

**Table 1**

| Compound (Example No.) | Minimum effective dose (µM) (A) | Cytotoxicity (µM)(B) | Selective toxicity (B/A) |
|---|---|---|---|
| 1 | 0.20 | 1.56 | 8 |
| 2 | 0.20 | 3.12 | 16 |
| 3 | 0.40 | 12.5 | 31 |
| 4 | 0.40 | 12.5 | 31 |
| 5 | 0.40 | 12.5 | 31 |
| 6 | 0.80 | 12.5 | 16 |

As shown in Table 1, the derivative (1) or pharmacologically acceptable salt thereof of the present invention is superior inducing activity of outgrowth of neurite to the known lactacystin with higher selective toxicity on the cells. Therefore novel effective lactacystin derivatives are provided.

The compounds of the present invention, as well as lactacystin, are the neurotrophic factor, and induce neuritogenesis and cause a transient increase in the intracellular cAMP level in mouse neuroblastoma cell line Neuro 2A. Therefore the lactacystin derivatives and salts thereof of the present invention can be used in the treatment of dementia. Accordingly the compounds of the present invention can be applied as a neurotrophic drug. The dosage is usually approximately 200 mg/man i.v. When the compounds of the present invention are administered in doses of 100 mg/kg i.p. in mice, no deaths are observed.

Following examples and referential examples illustrate the invention.

### Referential example 1

Lactacystin (45.5 mg) dissolved in ethanol (0.31 ml) and 0.1 N sodium hydroxide (0.93 ml) was stirred at room temperature for 30 minutes. Reaction mixture was neutralized with addition of 2N HCl and dried in vacuo. Residue was purified by preparative TLC (developer, THF : H₂ O = 10 : 1) to obtain γ-lactam 26.2 mg (yield : 94.0%).
¹H-NMR (270 MHz, CD₃ OD) δ 0.85 (d, J = 6.6 Hz, 3H, (CH₃)₂CH), 0.8 6 (d, J = 6.6 Hz, 3H, (CH₃)₂CH), 0.98 (d, J = 7.6 Hz, 3H, HOCHCHCH₃), 1.69 (m, 1H, (CH₃)₂CH), 2.86 (m, 1H, HOCHCHCH₃), 3.83 (d, J = 5.3 Hz, 1H, HOCH), 4.30 (d, J = 5.9 Hz, 1H, HOCHCHCH₃)
LRMS (FAB, Glycerol matrix) m/z 232 [(M+H)⁺ ; calcd for C₁₀H₁₈NO₅ : 232]

### Referential example 2

γ-lactam (43.3 mg, 0.187 mmol) was dissolved in dichloromethane (0.7 ml) under argon atmosphere. BOPCl (1.5 eq., 0.305 mmol. 77.6 mg) and triethylamine (3 eq., 0.609 mmol, 85µl) were added thereto. Reaction mixture was stirred at room temperature for 70 minutes. cooled to 0°C and added water therein to stop the reaction. The reaction mixture was transferred into separating funnel, extracted with chloroform and dried by adding Na₂SO₄. The extract was concentrated in vacuo and purified using silica gel column chromatography (extracting solvent; CHCl₃ : MeOH = 50 : 1) to obtain β-lactone 27.2 mg (yield : 68.0%).
¹H-NMR (270 MHz , CD₃Cl₃) δ 0.84 (d, J = 6.8 Hz, 3H, (CH₃)₂ CH), 0.99 (d, J = 6.8 Hz, 3H, (OH₃)₂CH), 1.27 (d, J = 7.6 Hz, 3H, CH₃CHCHOH), 1.8 2 (m, 1H, (CH₃)₂CH), 2.69 (m, 1H, CH₃CHCHOH), 3.91 (d, J = 6.9 Hz, 1H, HOCHCH), 5.15 (d, J = 6.3 Hz, 1H, CH₃CHCHOH), 6.25 (s, 1H, NH)
¹³ C-NMR (67.5 MHz, CDCl₃ δ 8.1, 16.3, 20.0, 29.6, 38.1, 64.0, 71.8, 76.0, 171.1, 176.8
LRMS (FAB, NBA matrix) m/z 214 [(M+H)⁺ ; calcd for C₁₀H₁₆NO₄ : 14]

### Example 1

β-lactone (10 mg, 0.047 mmol) obtained in the method of referential example 2 was dissolved in dichloromethane (1.0 ml) under argon atmosphere. Triethylamine (6 eq., 0.0282 mmol, 39.3µl) and ethanethiol (3 eq., 0.141 mmol. 10.4µl) were added thereto. Reaction mixture was stirred at 40 °C for 1 hour, concentrated in vacuo and purified using preparative TLC (developer; CHCl₃ : MeOH = 10 : 1) to obtain 3-hydroxy-2-(1-hydroxy-2-methylpropyl)-4-methyl-5-oxo-2-pyrrolidine-ethanethiocarboxylate 10.3 mg (yield : 80%).
¹H-NMR (270 MHz, CDCl₃) δ 0.79 (d, J = 6.6 H, 3H), 0.87 (d, J = 6.6 Hz, 3H), 0.9 8 (d, J = 7.6 Hz, 3H), 1.0 5 (t, J = 8.5 Hz - 3H), 1.58 (m, 1H), 2.25 (q, J = 8.5 Hz, 2H), 2.83 (m, 1H), 3.86 (d, J = 6.9 Hz, 1H), 4.43 (d, J = 6.6 Hz, 1H)
LRMS (FAB, NBA matrix) m/z 276 [(M+H)⁺; calcd for C₁₂H₂₂NO₄S : 276]

### Example 2

β-lactone (10 mg, 0.047 mmol) obtained in the method of referential example 2 was dissolved in dichloromethane (1.0 ml) under argon atmosphere. Triethylamine (6 eq., 0.0282 mmol, 39.3µl) and propanethiol (3 eq., 0.14 mmol, 14.5µl) were added thereto. Reaction mixture was stirred at 40 °C for 1 hour, concentrated in vacuo and purified using preparative TLC (developer: CHCl ₃: MeOH = 10 : 1) to obtain 3-hydroxy-2-(1-hydroxy-2-methylpropyl)-4-methyl-5-oxo-2-pyrrolidine-propanethiocarboxylate 10.6 mg (yield : 78%).
¹H-NMR (270 MHz, CDCl₃) δ 0.79 (d, J = 6.6 H, 3H), 0.87 (d, J = 6.6 Hz, 3H), 0.98 (d, J = 7.6 Hz, 3H), 1.05 (t, J = 8.5 Hz, 3H), 1.40 (m, 2H), 1.58 (m, 1H), 2.25 (q, J = 7.5 Hz, 2H), 2.83 (m, 1H) , 3.86 (d, J = 6.9 Hz, 1H), 4.43 (d, J = 6.6 Hz, 1H)
LRMS (FAB, NBA matrix) m/z 290 [(M+H)⁺; calcd for C₁₃H₂₄NO₄S : 290]

### Example 3

β-lactone (10 mg, 0.047 mmol) obtained in the method of referential example 2 was dissolved in dichloromethane (1.0 ml) under argon atmosphere. Triethylamine (6 eq., 0.0282 mmol, 39.3µl) and iso-propanethiol (3 eq., 0.141 mmol, 14.5 µl) were added thereto. Reaction mixture was stirred at 60°C for 3 hours, concentrated in vacuo and purified using preparative TLC (developer; CHCl ₃ : MeOH = 10 : 1) to obtain 3-hydroxy-2-(1-hydroxy-2-methylpropyl)-4-methyl-5-oxo-2-pyrrolidine-iso-propanethiocarboxylate 8.8 mg (yield : 65%).
¹H-NMR (270 MHz, CDCl₃) δ 0.79 (d, J = 6.6 H, 3H), 0.87 (d, J = 6.6 Hz, 3H), 0.98 (d, J = 7.6 Hz, 3H), 1.50 (d, J = 8.5 Hz, 6H), 1.58 (m, 1H), 2.53 (m, 1H), 2.83 (m, 1H), 3.86 (d, J = 6.9 Hz, 1H), 4.43 (d, 6.6 Hz, 1H)
LRMS (FAB, NBA matrix) m/z 290 [(M+H)⁺ ; calcd for C₁₃H₂₄NO₄S : 290]

### Example 4

β-lactone (10 mg, 0.047 mmol) obtained in the method of referential example 2 was dissolved in dichloromethane (1.0 ml) under argon atmosphere. Triethylamine (6 eq., 0.0282 mmol, 39.3µl) and butanethiol (3 eq., 0.14 mmol, 15.1 µl) were added thereto. Reaction mixture was stirred at 60°C for 5 hours, concentrated in vacuo and purified using preparative TLC (developer; CHCl₃ : MeOH = 10 : 1) to obtain 3-hydroxy-2-(1-hydroxy-2-methylpropyl)-4-methyl-5-oxo-2-pyrrolidine-butanethiocarboxylate 8.5 mg (yield : 62%).
¹H-NMR (270 MHz, CDCl₃) δ 0.79 (d, J = 6.6 H, 3H), 0.87 (d, J = 6.6 Hz, 3H), 0.90 (t, J = 8.5 Hz, 3H), 0.98 (d, J = 7.6 Hz, 3H), 1.20 ∼ 1.30 (m, 4H), 1.30 (m, 2H), 1.58 (m, 1H), 2.25 (q, J = 8.5 Hz, 2H), 2.83 (m, 1H), 3.86 (d, J = 6.9Hz, 1H), 4.43 (d, J = 6.6 Hz, 1H)
LRMS (FAB, NBA matrix) m/z 304 [(M+H)⁺; calcd C₁₄H₂₆NO₄ S : 304]

### Example 5

β-lactone (10 mg, 0.047 mmol) obtained in the method of referential example 2 was dissolved in dichloromethane (1.0ml) under argon atmosphere. Triethylamine (6 eq., 0.0282 mmol, 39.3µl) and 1-methyl-1-propanethiol (3 eq., 0.141 mmol, 10.4µl) were added thereto. Reaction mixture was stirred at 60°C for 15 hours, concentrated in vacuo and purified using preparative TLC (developer; CHCl₃ : MeOH = 20 : 1) to obtain 3-hydroxy-2-(1-hydroxy-2-methylpropyl)-4-methyl-5-oxo-2-pyrrolidine-1-methyl-l-propanethiocarboxylate 7.8 mg (yield : 55%).
¹H-NMR (270 MHz, CDCl₃) δ 0.79 (d, J = 6.6 H, 3H), 0.87 (d, J = 6.6 Hz, 3H), 0.98 (d, J=7. 6 Hz, 3H), 1.05 (d, J = 8.5 Hz, 6H), 1.40 (m, 1H), 1.58 (m, 1H), 2.25 (q, J = 8.5 Hz, 2H), 2.83 (m, 1H), 3.86 (d, J = 6.9 Hz, 1H), 4.43 (d, J = 6.6 Hz, 1H)
LRMS(FAB, NBA matrix) m/z 304 [(M+H)⁺ ; calcd for C₁₄H₂₆NO₄S : 304]

### Example 6

β-lactone (10 mg. 0.047 mmol) obtained in the method of referential example 2 was dissolved in dichloromethane (1.0 ml) under argon atmosphere. Triethylamine (6 eq., 0.0282 mmol. 39.3µl) and 1-pentanethiol (6 eq., 0.282 mmol, 35.0 µl) were added thereto. Reaction mixture was stirred at 40°C for 6 hours, concentrated in vacuo and purified using preparative TLC (developer; CHCl ₃ : MeOH = 20 : 1) to obtain 3-hydroxy-2-(1-hydroxy-2-methylpropyl)-4-methyl-5-oxo-2-pyrrolidine-1-pentanethiocarboxylate 10.4 mg (yield : 70%).
¹H-NMR (270 MHz, CDCl₃) δ 0.79 (d, J = 6.6 H, 3H), 0.80 (q, J = 8.5 Hz, 3H), 0.87 (d, J = 6.6 Hz, 3H), 0.98 (d, J = 7.6 Hz, 3H), 1.20 ∼ 1.40 (m, 6H), 1.58 (m, 1H), 2.25 (q, J = 8.5 Hz, 2H) 2.83 (m, 1H), 3.86 (d, J = 6.9 Hz, 1H), 4.43 (d, J = 6.6 Hz, 1H)
LRMS (FAB, NBA matrix) m/z 318 [(M+H) ⁺ ; calcd for C₁₅H₂₈NO₄S : 318]

## Claims

1. A compound which is lactacystin derivative of formula (I): 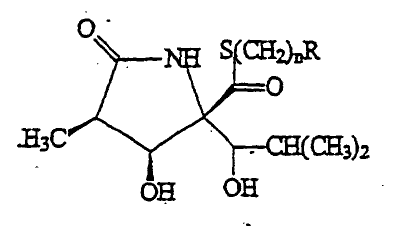 wherein:
R is branched or unbranched C₁-C₄ alkyl, and
n is 0 to 4,
or a pharmacologically acceptable salt thereof.

2. A compound according to claim 1, which is 3-hydroxy-2-(1-hydroxy-2-methylpropyl)-4-methyl-5-oxo-2-pyrrolidine-ethanethiocarboxylate, 3-hydroxy-2-(1-hydroxy-2-methylpropyl)-4-methyl-5-oxo-2-pyrrolidine-propanethiocarboxylate, 3-hydroxy-2-(1-hydroxy-2-methylpropyl)-4-methyl-5-oxo-2-pyrrolidine-iso-propanethiocarboxylate, 3-hydroxy-2-(1-hydroxy-2-methylpropyl)-4-methyl-5-oxo-2-pyrrolidine-butanethiocarboxylate, 3-hydroxy-2-(1-hydroxy-2-methylpropyl)-4-methyl-5-oxo-2-pyrrolidine-1-methyl-1-propanethiocarboxylate, 3-hydroxy-2-(1-hydroxy-2-methylpropyl)-4-methyl-5-oxo-2-pyrrolidine-1-pentanethiocarboxylate, or a pharmacologically acceptable salt thereof.

3. A compound as defined in claim 1 or claim 2 for use in a method of treatment of the human or animal body by therapy.

4. A compound as defined in claim 1 or claim 2 for use in inducing the outgrowth of neurites.

5. Use of a compound as defined in claim 1 or claim 2 in the manufacture of a medicament for inducing the outgrowth of neurites.

6. A process for the preparation of a compound as defined in claim 1 or claim 2 which comprises:
reacting β-lactone of formula (2): 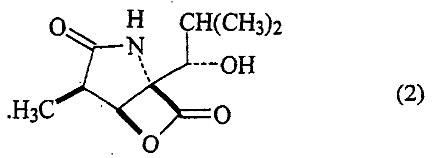 with a mercapto compound of formula HS-(CH₂)ₙR, in which n and R are as defined in claim 1, in the presence of a tertiary organic amine in an inert organic solvent.

7. A pharmaceutical composition which comprises a compound as defined in claim 1 or claim 2 and a pharmaceutically acceptable diluent or excipient

## Patentansprüche

1. Verbindung, nämlich ein Lactacystinderivat der Formel (I) 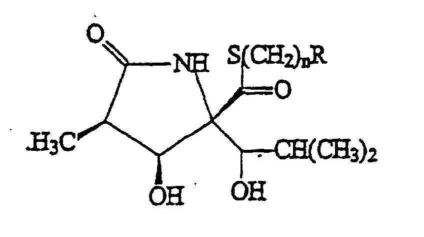 worin
R verzweigtes oder unverzweigtes C₁-C₄-Alkyl bedeutet und
n einen Wert von 0 bis 4 hat,
oder ein pharmakologisch verträgliches Salz davon.

2. Verbindung nach Anspruch 1, nämlich
3-Hydroxy-2-(1-hydroxy-2-methylpropyl)-4-methyl-5-oxo-2-pyrrolidin-ethanthiocarboxylat,
3-Hydroxy-2-(1-hydroxy-2-methylpropyl)-4-methyl-5-oxo-2-pyrrolidin-propanthiocarboxylat,
3-Hydroxy-2-(1-hydroxy-2-methylpropyl)-4-methyl-5-oxo-2-pyrrolidin-isopropanthiocarboxylat,
3-Hydroxy-2-(1-hydroxy-2-methylpropyl)-4-methyl-5-oxo-2-pyrrolidin-butanthiocarboxylat,
3-Hydroxy-2-(1-hydroxy-2-methylpropyl)-4-methyl-5-oxo-2-pyrrolidin-1-methyl-1-propanthiocarboxylat,
3-Hydroxy-2-(1-hydroxy-2-methylpropyl)-4-methyl-5-oxo-2-pyrrolidin-1-pentanthiocarboxylat,
oder ein pharmakologisch verträgliches Salz davon.

3. Verbindung nach Anspruch 1 oder 2 zur Verwendung in einem Verfahren zur therapeutischen Behandlung des menschlichen oder tierischen Körpers.

4. Verbindung nach Anspruch 1 oder 2 zur Verwendung bei der Induktion des Herauswachsens von Neuriten.

5. Verwendung einer Verbindung nach Anspruch 1 oder 2 bei der Herstellung eines Arzneimittels zur Induktion des Herauswachsens von Neuriten.

6. Verfahren zur Herstellung einer Verbindung nach Anspruch 1 oder 2, umfassend
das Umsetzen des β-Lactons der Formel (2) 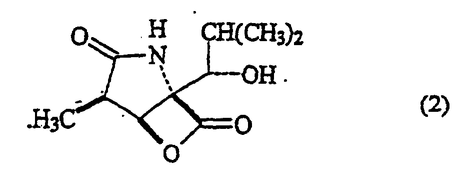 mit einer Mercaptoverbindung der Formel HS-(CH₂)ₙR, in der n und R die in Anspruch 1 definierten Bedeutungen haben, in Gegenwart eines tertiären organischen Amins in einem inerten organischen Lösungsmittel.

7. Pharmazeutische Zusammensetzung, enthaltend eine Verbindung nach Anspruch 1 oder 2 und ein pharmazeutisch verträgliches Verdünnungsmittel oder Exzipiens.

## Revendications

1. Composé qui est un dérivé de lactacystine de formule (I) : 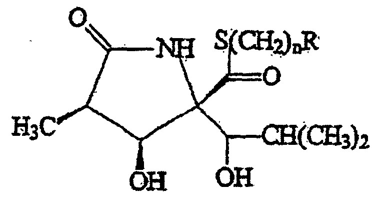 dans laquelle :
R est un groupe alkyle ramifié ou non ramifié en C₁-C₄ et
n vaut de 0 à 4,
ou un sel pharmacologiquement acceptable de celui-ci.

2. Composé selon la revendication 1, qui est du 3-hydroxy-2-(1-hydroxy-2-méthylpropyl)-4-méthyl-5-oxo-2-pyrrolidine-éthanethiocarboxylate, du 3-hydroxy-2-(1-hydroxy-2-méthylpropyl)-4-méthyl-5-oxo-2-pyrrolidine-propanethiocarboxylate, du 3-hydroxy-2-(1-hydroxy-2-méthylpropyl)-4-méthyl-5-oxo-2-pyrrolidine-isopropane-thiocarboxylate, du 3-hydroxy-2-(1-hydroxy-2-méthylpropyl)-4-méthyl-5-oxo-2-pyrrolidine-butanethiocarboxylate, du 3-hydroxy-2-(1-hydroxy-2-méthylpropyl)-4-méthyl-5-oxo-2-pyrrolidine-1-méthyl-1-propanethiocarboxylate, du 3-hydroxy-2-(1-hydroxy-2-méthylpropyl)-4-méthyl-5-oxo-2-pyrrolidine-1-pentanethiocarboxylate, ou un sel pharmacologiquement acceptable de celui-ci.

3. Composé tel que défini dans la revendication 1 ou la revendication 2, à utiliser dans un procédé de traitement du corps humain ou animal par thérapie.

4. Composé tel que défini dans la revendication 1 ou la revendication 2, à utiliser pour induire l'excroissance d'axones.

5. Utilisation d'un composé tel que défini dans la revendication 1 ou la revendication 2 pour la fabrication d'un médicament pour induire l'excroissance d'axones.

6. Procédé de préparation d'un composé tel que défini dans la revendication 1 ou la revendication 2, comprenant :
la mise en réaction d'une β-lactone de formule (2) : 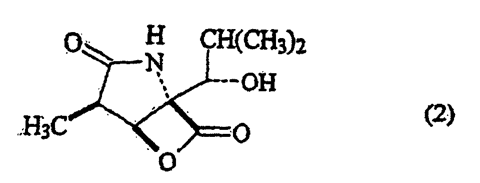 avec un composé mercapto de formule HS-(CH₂)ₙR, dans laquelle n et R sont tels que définis dans la revendication 1, en présence d'une aminé organique tertiaire dans un solvant organique inerte.

7. Composition pharmaceutique comprenant un composé tel que défini dans la revendication 1 ou la revendication 2 et un diluant ou un excipient pharmaceutiquement acceptable.
